# EUROPEAN PATENT SPECIFICATION

(11) **EP 2 207 036 B1**
(45) Date of publication and mention of the grant of the patent: **12.12.2012**
(21) Application number: 10157543.9
(22) Date of filing: 24.03.2004
(51) Int. Cl.: G01N 33/569, G01N 33/543, G01N 33/53

(54) **Methods for determining the negative control value for multi-analyte assays**
Methoden zur Messung der Negativkontrolle in Mehrfachanalyt-Assays
Procédé de determination de témoins négatifs pour essais multi-analytes

(30) Priority: 24.03.2003 US 457146 P
(43) Date of publication of application: 14.07.2010
(62) Divisional of application: 04251704.5
(73) Proprietor: Gen-Probe Transplant Diagnostics, Inc., Stamford, CT 06902 (US)
(72) Inventor: Nordman, David, Metairie, LA 700002 (US); Ray, Bryan, Cheshire, CT 06410 (US); Balazs, Ivan, New Rochelle, NY 10804 (US)
(74) Representative: Clarke, Lionel Paul

(56) References cited:
- US-A- 5 962 239
- US-A- 6 046 013
- US-A1- 2003 017 447

## Description

### Field of the Invention

This invention relates to methods for determining the negative control value for multi-analyte assays.

### Background of Invention

Various analytical procedures and devices are commonly employed in assays to determine the presence and/or concentration of substances of interest or clinical significance that may be present in biological liquids or other materials. Such substances are commonly termed "analytes" and can include antibodies, antigens, drugs, hormones, etc.

One frequently used assay format is the immunoassay. Immunoassay techniques take advantage of the mechanisms of the immune systems of higher organisms, wherein antibodies are produced in response to the presence of antigens that are pathogenic or foreign to the organisms. These antibodies and antigens, i.e., immunoreactants, are capable of binding with one another, thereby creating a highly specific reaction mechanism that can be used *in vitro* to determine the presence or concentration of a particular antigen or antibody in a biological sample.

There are several known immunoassay methods using immunoreactants, wherein at least one of the immunoreactants is labeled with a detectable component so as to be analytically identifiable. For example, the "sandwich" or "two-site" technique may involve the formation of a ternary complex between an antigen and two antibodies. A convenient method of detecting the presence of analytes in a sample is to provide an unlabeled antibody bound to a solid phase support such that the complex can readily be formed between the unlabeled antibody and the analyte present in the sample. After washing, the solid support is then contacted with labeled antibodies that also bind to the analyte. In this example, the amount of labeled antibody associated with the solid phase is directly proportional to the amount of analyte in the test sample.

In a similar assay format, an antigen may be bound to a solid support, which is then contacted with a test solution that may or may not contain antibodies to the antigen. After washing, the solid support is then contacted with labeled antibodies that bind to any antibodies bound to the analyte. The labeled antibodies can then be detected and their presence directly correlated with the presence of the analyte.

An alternative technique is the "competitive" assay. In one example of a competitive assay, the capture mechanism again may use an antibody attached to an insoluble solid phase, but a labeled analyte competes with the analyte present in the test sample for binding to the immobilized antibody. Similarly, an immobilized analyte can compete with the analyte of interest for a labeled antibody. In these competitive assays, the quantity of captured labeled reagent is inversely proportional to the amount of analyte present in the sample.

A multi-analyte system should involve a means of providing simultaneous analysis of several analytes in a test sample. This analysis should provide results that identify individual analytes and enable the quantitation of each individual analyte in that test sample. A method of multi-analyte analysis is often claimed but the given criteria are generally not both fulfilled.

In a multi-analyte system, a typical substrate contains a plurality of individual test reaction sites each possessing a different binding ligand. The test sample contacts each of the reaction zones and thereafter a variety of detection techniques can be implemented to identify the analyte(s) present. It is preferable that the detection method used enables quantitation of each individual analyte.

Conventional assay methods often use the values obtained from the reaction of a negative control biological material (e.g. sera) with the muiti-analyte panel. Often, multiple negative control sera are used. These negative control sera, obtained from several individuals and different from those providing the sample sera, are used to correct for non-specific reaction of sera in the multi-analyte assay. Problems may arise due to the fact that the source of the negative samples is different from that of the unknown sample, resulting in unexpected reactivity.

Thus, conventional methods of determining the negative control value may produce an inaccurate estimate of the true negative value. As a result, for some samples, the multi-analyte assay may produce false positive or false negative results.

### Brief Summary

The present invention provides unique and advantageous methods for selecting the negative control value to be used when correcting the results obtained from measuring the reaction of a complex biological mixture in a multi-analyte assay.

The methods of the subject invention are particularly advantageous because the negative control value(s) is/are generated using the same sample that is also being analyzed for the presence of the analytes of interest.

We also describe testing of a blood serum sample using indirect detection methods for immunofluorescence or ELISA type assays. This method provides for a negative control value that, when incorporated into sample calculations, reliably determines whether the unknown sera contains specific antibodies against any of the antigens bound to a solid support.

### Detailed Disclosure

The subject invention advantageously provides new and reliable methods for selecting the negative control value(s) to be used in multi-analyte assays as defined in the claims. Use of the methods of the subject invention can reduce the incidence of false positives and/or false negatives, particularly in multi-analyte assays conducted on complex biological samples.

Advantageously, in accordance with the practice of the subject invention, the negative control value(s) can be selected from the results obtained only with the sample biological material (e.g. patient sera), without the need for processing negative control material (e.g. normal sera).

We also describe utilizing one or more "negative reagents" as the binding ligand in the assay. As used herein, "binding ligand" or "capture reagent" refers to an entity attached to a solid support and which specifically binds to a target analyte, except for the negative control reagent, which is a reagent for which it is known that the sample of interest does not contain a chemical entity that would specifically bind to the reagent. Thus, this negative control reagent could be, for example, an antigen for which it is known that the sample of interest does not contain an antibody that specifically binds to the antigen. For example, a non-human HLA protein could be used as the negative control reagent for a test wherein the sample of interest is a human sample. Preferably, the negative control reagent is an entity that has physical, chemical, and/or antigenic properties in common with at least one capture reagent. The common properties may be, for example, molecular weight, charge, and/or solubility. The negative control reagent may be a homolog, ortholog or other such related molecule to the capture reagent.

It is, of course, ideal to find a negative control reagent that generally behaves the same as the reagents used to detect the specific target analyte(s). However, with any biological assay, there is always the possibility of encountering samples having non-specific variable interactions that pass unrecognized and affect the interpretation of the assay. Thus, to reliably interpret the results of any assay, it is advantageous to not only have within one's repertoire the use of negative control reagents, but multiple techniques for determining negative control values (NCVs).

The methods of the subject invention can be used to determine an appropriate NCV even without utilizing either traditional negative control sera or the negative control reagent system as described herein. Reactivity values for one or more low-reacting specific target analytes in a multi-analyte assay are used to establish the NCV(s).

The methods of the subject invention can be applied to the testing of a blood serum sample using indirect detection methods for immunofluorescence or ELISA type assays. This method provides for identification of a NCV that, when incorporated into sample calculations, reliably determines whether an unknown sera contains specific antibodies against any of the capture reagents bound to a solid support. The solid support may be, for example, beads, wells, membranes, or microarrays.

In one specific embodiment of the subject invention, the panel of binding ligands includes, in addition to the capture reagents for the specific target analytes, one or more non-human proteins or other compounds to neutralize possible charge or chemical interactions that may occur with the solid support. Examples of this type of compound include proteins such as albumin or casein or chemicals that bind or react with the solid support; for example, if the solid support is a carboxylated plastics, it is possible to use chemicals containing amino groups such as ethanolamine or tris (hydroxymethyl) aminomethane.

Following are Examples that illustrate procedures for practising the invention. These examples should not be construed as limiting. All percentages are by weight and all solvent mixture proportions are by volume unless otherwise noted.

### Example 1 - Screening for anti-HLA antibodies in human sera

One or more Human Leukocyte Antigens (HLAs) are attached to a solid surface (e.g. multiple wells of a microtiter plate, or different color or size beads) creating a panel of HLAs. This panel of multiple wells or beads also includes negative controls consisting of one or more wells or beads with molecules that are not HLAs. Patient sera is added to all the wells, or mixed with the different beads, and incubated. After washing, the presence of anti-HLA antibodies can be measured by reaction to a labeled anti-immunoglobulin antibody.

Conventional methods produce a negative control by performing several assays using sera from individuals that do not contain anti-HLA antibodies. This is done in order to estimate the amount of non-specific reactivity between sera and capture reagents (antigens) on the solid support. Typically, the median or average values are used to establish a negative control value (NCV).

US-A-6046013 describes a typical process for obtaining the negative control value using a similar type of assay.

It would be extremely rare for an individual to have anti-HLA antibodies against every antigen in the panel; therefore, in one embodiment of the subject invention, the antibody/antigen complex with the lowest signal can be considered the raw NCV. There may be several analytes with similar values; however, since additional calculations will be performed to compensate for assay variability, in one embodiment it is sufficient to use the lowest value. Alternatively, an average of multiple low values can be used. These "low" values would be clearly distinguishable from higher values that reflect a positive result for a particular analyte. The "low" values would, for example, be within 20% of the lowest value and, preferably, within 10% of the lowest value.

The raw NCV may be further adjusted by subtracting the background value (BGV) (i.e. inherent reactivity of the solid support) to obtain a calculated NCV.

The BGV reflects the inherent reactivity of each capture reagent, with the detection antibody. This can be measured by adding water or buffer instead of serum sample to the multi-analyte panel. The BGV may be the same or vary significantly between capture reagents. If there are large differences between the BGVs, each analyte measurement can be corrected by subtracting its own BGV. Alternatively, it is possible to use the same median BGV or average BGV for all analytes.

Another way to determine the NCV for non-specific binding of sample sera to the solid support is by evaluating the reactivity of the sample with one or more non-human HLA-like proteins. In a specific embodiment, leukocyte antigens from non-primates can be attached to a solid support to create non-specific interactions similar to those produced by HLA.

### Example 2 - Determination of Negative Control Value

Table 1 shows how to identify the NCV in accordance with one embodiment of the methods of the subject invention.

An "analyte panel" was created by attaching 17 capture reagents to a solid support. A positive (#16) and negative (#17) control were included on the panel.

The sample and water were contacted with the analyte panel (the panel of capture reagents) and subsequently rinsed to wash away unbound molecules. A label was added to detect the attached molecules. The label was detected and the intensity or amount (e.g., absorbance, fluorescence) recorded as shown in Table 1.

The value recorded from each reaction of water and capture reagent is an individual BGV. The average BGV of 26 could be used, but the BGVs had a broad range; therefore, each individual BGV was subtracted from the raw value recorded from each reaction of the sample. These adjusted values were compared to the negative control (#17).

A review of Table 1 reveals that the lowest adjusted value was the reaction of the sample with capture reagent #4 (123) instead of the negative control #17 (530). Therefore, the value for analyte #4 can be used as the negative control instead of the value for analyte #17. Otherwise other analytes would end up as false negatives.

In this example, the raw NCV is the raw value of the sample reacted with capture reagent #4 (158). The calculated NVC is identified by subtracting the raw value of water reacted with capture reagent for analyte #4 (35) from the raw value of the sample reacted with the capture reagent for analyte #4 (158). Therefore, the NCV is 123.

**Table 1.**

| The "analyte panel" contains capture reagents for the detection of 15 different analytes plus one positive and one negative control. | | | | | | | | | | | | | | | | | | |
|---|---|---|---|---|---|---|---|---|---|---|---|---|---|---|---|---|---|---|
| | Analyte panel > | Analyten #1 | 2 | 3 | 4 | 5 | 6 | 7 | 8 | 9 | 10 | 11 | 12 | 13 | 14 | 15 | 16 | 17 |
| Water BG: | Individual BG Values > | 36 | 11.5 | 12 | 35 | 24 | 10 | 89 | 32 | 5 | 6 | 1 | 33 | 13 | 51 | 10 | 17.5 | 54 |
| Sample: | Values w/each analyte > | 291 | 166 | 443 | 158 | 296 | 300 | 495 | 543 | 595 | 847 | 2549 | 1568 | 356 | 461 | 271 | 1500 | 584 |
| raw NCV | | | | | | | | | | | | | | | | | | |
| Sample: | Adjusted Values > | 255 | 154 | 431 | 123 | 272 | 290 | 406 | 511 | 590 | 841 | 2548 | 1535 | 343 | 410 | 261 | 1483 | 530 |
| calc NCV | | | | | | | | | | | | | | | | | | |
| Average BG = | 26 | (not used in this example) | | | | | | | | | | | | | | | ^ positive control | ^ negative control |

### Example 3 -Determination of Negative Control Value

Table 2 shows another example of how to identify the NCV in accordance with the methods of the subject invention.

The same panel for the detection of 17 analytes used in Example 2 was used in this example. This panel included a positive (#16) and a negative (#17) control. However, a different sample was used in this example. In this case, the lowest adjusted value was that obtained from the reaction of the sample with the negative control #17. Therefore, in this case, the adjusted value for #17 would be used as the NCV. Otherwise, many analytes would end up as false negatives.

**Table 2.**

| The "analyte panel" contains capture reagents for the detection of 15 different analytes plus one positive and one negative control. | | | | | | | | | | | | | | | | | | |
|---|---|---|---|---|---|---|---|---|---|---|---|---|---|---|---|---|---|---|
| | Analyte panel > | Analyte #1 | 2 | 3 | 4 | 5 | 6 | 7 | 8 | 9 | 10 | 11 | 12 | 13 | 14 | 15 | 16 | 17 |
| Water BG: | Individual BG Values > | 36 | 11.5 | 12 | 35 | 24 | 10 | 89 | 32 | 5 | 6 | 1 | 33 | 13 | 51 | 10 | 17.5 | 54 |
| Sample: | Values w/each analyte > | 291 | 166 | 443 | 158 | 296 | 300 | 495 | 543 | 595 | 847 | 2549 | 1568 | 456 | 461 | 471 | 1500 | 84 |
| raw NC | | | | | | | | | | | | | | | | | | |
| Sample: | Adjusted Values > | 255 | 154 | 431 | 123 | 272 | 290 | 406 | 511 | 590 | 841 | 2548 | 1535 | 443 | 410 | 461 | 1483 | 30 |
| calc NC | | | | | | | | | | | | | | | | | | |
| Average BG = | 26 | (not used in this example) | | | | | | | | | | | | | | | ^ positive control | ^ negative control |

### Example 4

Conventional methods utilize a single "negative reagent" to determine the level of non-specific interaction of a sample in a multi-analyte assay. This is done in order to estimate the amount of non-specific reactivity between sera and the multi-analyte solid support. However, with any biological assay, there is always the possibility of encountering samples having non-specific variable interactions. These interactions may occur with one "negative reagent" but not with a chemically different "negative reagent". Thus, it is advantageous to have within one's repertoire multiple types of "negative reagent" controls (NRC).

In one preferred embodiment, two or more different NRC are included in a multi-analyte panel. After the assay with the sample sera, a calculated NC value is obtained for each NRC. The calculated NC value for each NRC is used separately to evaluate the reactivity of the sample against the analytes in the panel. This produces as many sets of results as the number of NRCs included in the multi-analyte panel. The comparison of these sets shows which analytes are consistently positive or negative with the different NRCs.

For example, if four independent NRCs are included in the multi-analyte panel, four sets of results are generated. By counting the number of times that an analyte generates the same type of results in the four sets, it is possible to determine the concordance or consistency of the results.

One method to interpret the results is to count the number of times an analyte is positive in the four sets. If the analyte is positive in 3 or 4 sets of results, it would be considered Positive. If it was positive in 2 sets it would be considered as Tentative or Doubtful Positive. If it was positive in 1 set it would be considered as Tentative or Doubtful Negative. If it was not positive in any of the sets in would be considered Negative.

### Example 5 - Effect of Multiple Control beads on Results from an Antibody Screening Assay

A serum sample was assayed using the Lifematch Antibody Screen assay (Tepnel Lifecodes, Stamford, CT), designed for use with a Luminex 100 fluoranalyzer, which was modified to include three negative control beads. This product consists of one bead population with HLA Class I antigens attached, a second population with Class II antigens attached, a third population with Human IgG that serves as a positive control for the detection reagent, a fourth population that contains an analyte not related to HLA that serves as negative control (CON1), a fifth population that contains a different analyte as a second negative control (CON2) and a sixth population that contains yet a different analyte that serves as a third negative control (CON3).

As described below, the median fluorescent intensity (MFI) for each antigen-containing bead (i.e., Class I or Class II beads) is divided by the MFI of each negative control bead yielding 3 quotients. From each quotient is subtracted a background adjustment factor (BAF) that is provided by the kit manufacturer. This results, for each analyte, in three *adjusted values* (AdjVal). If any adjusted value is greater than zero, a positive reactivity for an antigen-containing bead is indicated. Table 3 illustrates the results obtained for this example assay.

**Table 3**

| Bead | MFI | BAF | AdjVal1 | AdjVal2 | AdjVal-3 | Result |
|---|---|---|---|---|---|---|
| | | | (a) | (b) | (c) | |
| Class I | 10,137.5 | | 52.92 | 29.94 | 28.97 | Positive |
| CON1 | 182 | 2.785 | | | | |
| CON2 | 311.5 | 2.608 | | | | |
| CON3 | 323.5 | 2.367 | | | | |
| Class II | 652 | | 0.51 | -0.92 | -0.76 | Positive |
| CONI | 182 | 3.068 | | | | |
| CON2 | 311.5 | 3.009 | | | | |
| CON3 | 323.5 | 2.774 | | | | |

| | | | | | | |
|---|---|---|---|---|---|---|
| (a) Adjval1 = (MFI/CON1) - BAF_{CON 1;} (b) AdjVal2 = (MFI/CON2) - BA_{CON 2;} (c) AdjVal3 = (MFI/CON3) - BAF_{CON 3} | | | | | | |

For Class I, a positive reaction is indicated by all three adjusted values, whereas for Class II, a positive reaction is indicated for only one adjusted value. Because two of the negative control beads (CON2 & CON3) show a higher background than CON1, they yield a false negative result.

As shown in this example, if only CON2 or CON3 were present in the assay, this sample would have been incorrectly assigned as having no antibody toward Class II antigens. Thus, inclusion of multiple negative control beads reduces the likelihood of a false negative assignment.

### Example 6 - Application of the lowest signal analyte as Negative Control to compensate for effect of high background

This example illustrates the effect of employing the lowest signal analyte as negative control for the analysis of a sample that has a high background with the Negative Control Bead.

A serum sample containing antibodies toward HLA-A2 and HLA-A68 antigens was assayed using the Lifematch Class I ID kit (Tepnel Lifecodes, Stamford, CT) and analyzed with a Luminex 100 fluoroanalyzer. The assay was performed according to the product insert. Briefly, the product contains a Negative Control Bead, a Positive Control Bead and a series of beads that contain various combinations of antigens. The assay comprises 1) mixing a serum sample with the beads, 2) washing away unbound sera, 3) adding a label that binds to the captured HLA-antibodies and 4) detecting the amount of label bound to each bead with the Luminex 100. The amount of label bound is reported as median fluorescence intensities (MFI).

For this example, the MFI values for each analyte is shown in Table 4. The results show that this sample has a high non-specific interaction with the Negative Control (CON1) bead.

In one method of analysis, the MFI for each antigen-containing bead is divided by the MFI of CON1. From this quotient is subtracted a background adjustment factor (BAF) that is provided by the kit manufacturer. A resultant greater than zero indicates a positive reactivity for an antigen-containing bead.

The use of this calculation for CON1 results in the classification of only 2 analyte containing beads as positive. As such, the sample may be erroneously classified as antibody negative. In addition, the concordance between the positive beads and a particular analyte (e.g. HLA-A antigen) in the bead is insufficient for antibody identification.

An alternative method for calculating a positive reaction employs an antigen-containing bead as a negative control. For most assays, a serum sample will not have a positive reaction with all antigen-containing beads. Thus, the antigen-containing bead that has the lowest MFI reading can be employed as a negative control to calculate an adjusted value (AdjVal2).

To determine reactivity, the same calculation is applied to the antigen-containing beads except that the MFI of CON1 bead is replaced by the MFI of the lowest value bead. In this same example, using the MFI of the lowest value bead (#156), results in the classification of 26 analyte containing beads, as positive (AdjVal2; Table 4). An examination of the analytes present in those beads shows 100% correlation with the presence of HLA-A2 and HLA-A68 on the beads. Therefore, in samples with high values for CON1, the analysis of the results using the lowest value analyte containing bead, allows for the correct identification of the antibodies present in the sample.

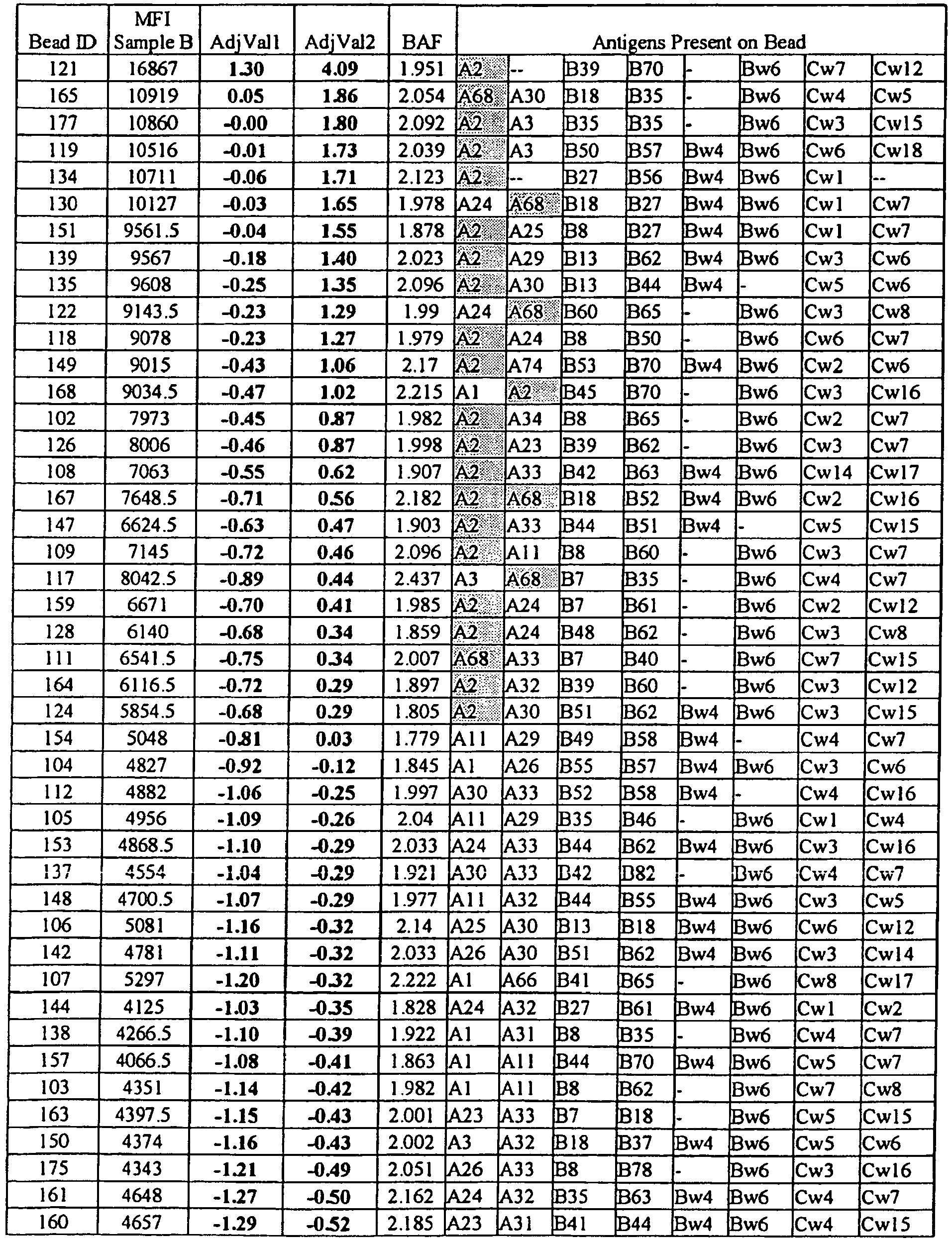

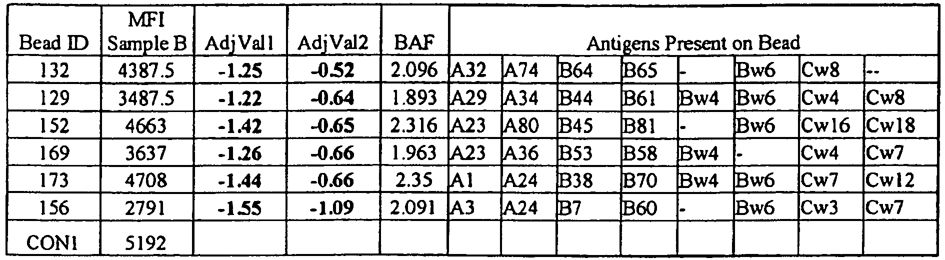

The following constitute preferred numbered embodiments according to the present invention:

## Claims

1. A multi-analyte assay for determining a presence or absence of analytes in a sample, wherein the analytes are antibodies, and wherein said assay comprises the following steps:
(a) contacting a sample with a plurality of human leukocyte antigens as capture reagents;
(b) measuring the reaction of the sample with each capture reagent;
(c) determining the presence or absence of analytes in the sample using, as a negative control value, the lowest value of the measured reactions or an average of multiple low values of the measured reactions, wherein, for the average of multiple low values, the low values are within 20% of the lowest value; and
(d) comparing the results of the reaction with the capture reagents and the negative control value to determine the presence or absence of the analytes in the sample.

2. The assay according to claim 1, wherein the capture reagents further include non-human leukocyte antigens.

3. The assay according to claim 1 or claim 2, wherein the capture reagents are attached to a solid support selected from the group consisting of beads, wells, membranes and microarrays.

4. The assay according to claim 1 or claim 2, wherein the sample is a serum, tissue, or urine sample.

5. The assay, according to claim 2, wherein at least one of the capture reagents is a non-primate leukocyte antigen.

## Patentansprüche

1. Multi-Analyt-Assay zur Bestimmung des Vorhandenseins oder des Fehlens von Analyten in einer Probe, wobei die Analyte Antikörper sind und wobei dieses Assay die folgenden Schritte umfasst:
(a) In Kontakt Bringen einer Probe mit einer Vielzahl von humanen Leukozyten-Antigenen als Fangreagenzien;
(b) Messen der Reaktion der Probe mit jedem Fangreagenz;
(c) Bestimmung des Vorhandenseins oder des Fehlens von Analyten in der Probe unter Verwendung des niedrigsten Werts der gemessenen Reaktionen oder eines Mittelwerts von mehreren niedrigen Werten der gemessenen Reaktionen als negativer Kontrollwert, wobei die niedrigen Werte für den Mittelwert der mehreren niedrigen Werte innerhalb von 20 % des niedrigsten Werts liegen, und
(d) Vergleich der Ergebnisse der Reaktion mit den Fangreagenzien und der negativen Kontrollwerte, um das Vorhandensein oder das Fehlen der Analyte in der Probe zu bestimmen.

2. Assay nach Anspruch 1, wobei die Fangreagenzien des Weiteren nichthumane Leukozyten-Antigene umfassen.

3. Assay nach Anspruch 1 oder Anspruch 2, wobei die Fangreagenzien an einen festen Träger gebunden sind, der aus der Gruppe bestehend aus Perlen, Wells, Membranen und Mikroarrays ausgewählt wird.

4. Assay nach Anspruch 1 oder Anspruch 2, wobei die Probe ein Serum, ein Gewebe oder eine Urinprobe ist.

5. Assay nach Anspruch 2, wobei mindestens eines der Fangreagenzien ein nicht von Primaten stammendes Leukozyten-Antigen ist.

## Revendications

1. Essai multi-analyte pour déterminer la présence ou l'absence d'analytes dans un échantillon, dans lequel les analytes sont des anticorps et dans lequel ledit essai comprend les étapes suivantes consistant à :
(a) mettre en contact un échantillon avec une pluralité d'antigènes de leucocytes humains comme réactifs de capture ;
(b) mesurer la réaction de l'échantillon avec chaque réactif de capture ;
(c) déterminer la présence ou l'absence d'analytes dans l'échantillon en utilisant, comme valeur des témoins négatifs, la valeur la plus basse des réactions mesurées ou une moyenne de multiples valeurs basses des réactions mesurées, dans lequel, pour la moyenne des multiples valeurs basses, les valeurs basses se situent dans les 20 % de la valeur la plus basse ; et
(d) comparer les résultats de la réaction avec les réactifs de capture et la valeur des témoins négatifs pour déterminer la présence ou l'absence des analytes dans l'échantillon.

2. Essai selon la revendication 1, dans lequel les réactifs de capture comprennent par ailleurs des antigènes leucocytaires non humains.

3. Essai selon la revendication 1 ou la revendication 2, dans lequel les réactifs de capture sont fixés à un support solide choisi dans le groupe constitué de billes, de puits, de membranes et de microréseaux.

4. Essai selon la revendication 1 ou la revendication 2, dans lequel l'échantillon est un échantillon de sérum, de tissu ou d'urine.

5. Essai selon la revendication 2, dans lequel au moins l'un des réactifs de capture est un antigène leucocytaire de non-primate.
